(19) Europäisches Patentamt
European Patent Office
Office européen des brevets

(11) Veröffentlichungsnummer: **0 150 040 B1**

(12) # EUROPÄISCHE PATENTSCHRIFT

(45) Veröffentlichungstag der Patentschrift: **31.08.94**

(21) Anmeldenummer: **85100381.4**

(22) Anmeldetag: **16.01.85**

Die Akte enthält technische Angaben, die nach dem Eingang der Anmeldung eingereicht wurden und die nicht in dieser Patentschrift enthalten sind.

(51) Int. Cl.5: **C07D 487/14**, C07D 487/04, C07D 495/14, C07D 495/22, A61K 31/555, //C07D243/14, C07D495/04,(C07D487/14, 243:00,235:00,203:00), (C07D487/04,243:00,235:00), (C07D487/14,243:00,235:00, 209:00),(C07D495/14,333:00, 243:00,235:00)

(54) **Imidazodiazepin-Derivate.**

(30) Priorität: **19.01.84 CH 225/84**
**29.06.84 CH 3149/84**
**26.10.84 CH 5123/84**

(43) Veröffentlichungstag der Anmeldung:
**31.07.85 Patentblatt 85/31**

(45) Bekanntmachung des Hinweises auf die Patenterteilung:
**31.08.94 Patentblatt 94/35**

(84) Benannte Vertragsstaaten:
**AT BE CH DE FR GB IT LI LU NL SE**

(56) Entgegenhaltungen:
**EP-A- 0 027 214**
**EP-A- 0 100 906**
**EP-A- 0 109 921**

(73) Patentinhaber: **F. HOFFMANN-LA ROCHE AG**
**Postfach 3255**
**CH-4002 Basel (CH)**

(72) Erfinder: **Hunkeler, Walter, Dr.**
**Im Stigler 32**
**CH-4312 Magden (CH)**
Erfinder: **Kyburz, Emilio, Dr.**
**Unterer Rebbergweg 127**
**CH-4153 Reinach (CH)**

(74) Vertreter: **Lederer, Franz, Dr.**
**Lederer, Keller & Riederer**
**Patentanwälte**
**Prinzregentenstrasse 16**
**D-80538 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die vorliegende Erfindung betrifft neue Imidazodiazepin-Derivate. Im speziellen betrifft sie Imidazodiazepin-Derivate der allgemeinen Formel

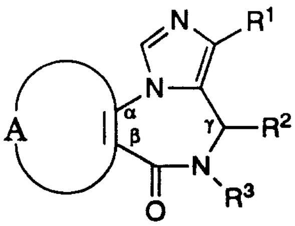

I

worin A zusammen mit den beiden mit α und β bezeichneten Kohlenstoffatomen eine der Gruppen

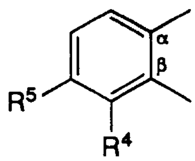

(1)

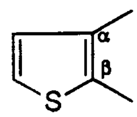

(2)

und

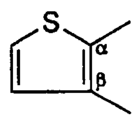

(3)

und worin $R^1$ 3-Cyclopropyl-1,2,4-oxadiazol-5-yl oder 5-Cyclopropyl-1,2,4-oxadiazol-3-yl, $R^2$ Wasserstoff und $R^3$ $C_1$-$C_7$-Alkyl oder $R^2$ und $R^3$ zusammen Dimethylen, Trimethylen oder Propenylen und $R^4$ und $R^5$ je Wasserstoff oder Halogen bedeuten, wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen, Trimethylen oder Propenylen bedeuten,
und pharmazeutisch annehmbare Säureadditionssalze davon, welche wertvolle pharmakodynamische Eigenschaften besitzen.

In der EP-A-109921 werden Verbindungen beschrieben welche der Formel I entsprechen, jedoch anstelle einer Cyclopropylgruppe am Oxadiazol-Ring eine niedere Alkylgruppe besitzen.

Ähnliche Verbindungen sind ferner aus EP-A-0027214 und EP-A-0100906 bekannt.

Gegenstand der vorliegenden Erfindung sind Verbindungen der obigen Formel I und pharmazeutisch annehmbare Säureadditionssalze davon als solche und als pharmazeutische Wirkstoffe, die Herstellung dieser Verbindungen und Zwischenprodukte für deren Herstellung, ferner Arzneimittel, enthaltend eine Verbindung der Formel I oder ein pharmazeutisch annehmbares Säureadditionssalz davon, die Herstellung solcher Arzneimittel und die Verwendung der erfindungsgemässen Stoffe bei der Bekämpfung oder Verhütung von Krankheiten.

"$C_1$-$C_7$-Alkyl", bezeichnet geradkettige oder verzweigte, gesättigte Kohlenwasserstoffreste, wie Methyl, Aethyl, n-Propyl, Isopropyl, n-Butyl, s-Butyl, t-Butyl.

Vorzugsweise bedeuten $R^2$ Wasserstoff und $R^3$ C1-$C_7$-Alkyl oder $R^2$ und $R^3$ zusammen Dimethylen oder Trimethylen. Wenn $R^2$ und $R^3$ zusammen Dimethylen oder Trimethylen bedeuten, so weisen die entsprechenden Verbindungen der Formel I bezüglich des mit α bezeichneten Kohlenstoffatoms vorzugsweise die (S)-Konfiguration auf.

Ein im Rahmen der vorliegenden Erfindung ganz besonders bevorzugter Vertreter der durch die allgemeine Formel I definierten Stoffklasse ist:

7-Chlor-3-(3-cyclopropyl-1,2,4-oxadiazol-5-yl)-5,6-dihydro-5-methyl-4H-imidazol[1,5-a][1,4]-benzodiazepin-6-on.

Weitere besonders bevorzugte erfindungsgemässe Verbindungen der Formel I sind:

(S)-8-Chlor-1-(3-cyclopropyl-1,2,4-oxadiazol-5-yl)-12,12a-dihydro-9H,11H-azeto[2,1-c]imidazo[1,5-a][1,4]-benzodiazepin-9-on,

(S)-8-Chlor-1-(3-cyclopropyl-1,2,4-oxadiazol-5-yl)-11,12,13,13a-tetrahydro-9H-imidazo[l,5-a]pyrrolo[2,1-c][1,4]benzodiazepin-9-on und

3-(3-Cyclopropyl-1,2,4-oxadiazol-5-yl)-8-fluor-5,6-dihydro-5-methyl-4H-imidazo[l,5-a][1,4]benzodiazepin-6-on.

Die Verbindungen der obigen Formel I und ihre pharmazeutisch annehmbaren Säureadditionssalze können ausgehend von den an sich bekannten Verbindungen der allgemeinen Formeln

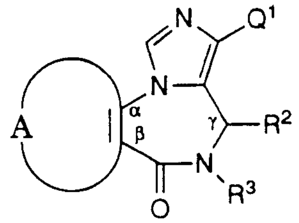
IIa

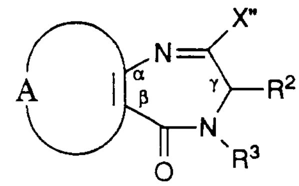
V

worin $Q^1$ die Gruppe -C(H) = NOH, -C≡N, $-CONH_2$ oder -COX und X und X" Abgangsgruppen bedeuten und $R^2$, $R^3$ und A obige Bedeutung besitzen, nach an sich bekannten und jedem Fachmann geläufigen Methoden hergestellt werden. Die im Hinblick auf die gewünschte Verbindung der Formel I zu treffende Auswahl der geeigneten Ausgangsstoffe, Reagenzien und Reaktionsbedingungen bietet dem Fachmann keinerlei Schwierigkeiten.

Im speziellen können Verbindungen der Formel I ausgehend von Verbindungen der allgemeinen Formel

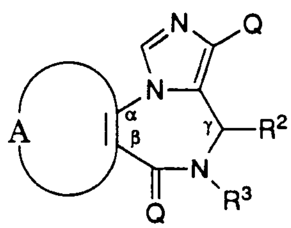
II

worin $R^2$, $R^3$ und A obige und Q die nachstehend angegebene Bedeutung besitzen, dadurch hergestellt werden, dass man

a) eine Verbindung der Formel II, worin Q die Gruppe

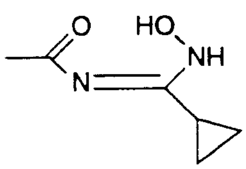
(a) ,
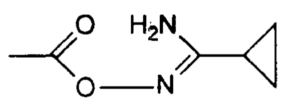
(b) oder
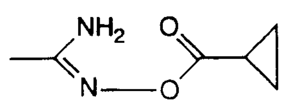
(c)

bedeutet, cyclisiert, oder

b) eine Verbindung der Formel II, worin Q die Gruppe -C≡N→O (d) bedeutet, mit Cyclopropylnitril oder eine Verbindung der Formel II, worin Q die Gruppe -CN (e) bedeutet, mit Cyclopropylnitriloxid umsetzt, oder dadurch, dass man

c) eine Verbindung der allgemeinen Formel

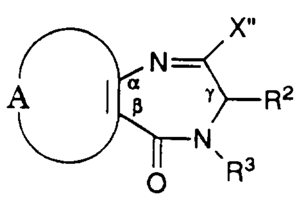
V

worin X" eine Abgangsgruppe bedeutet und $R^2$, $R^3$ und A obige Bedeutung besitzen, in Gegenwart einer Base mit einem Isonitril der allgemeinen Formel

$CN-CH_2-R^1$ XX

worin $R^1$ obige Bedeutung besitzt,
umsetzt; und wonach man
d) erwünschtenfalls eine erhaltene Verbindung der Formel I in ein pharmazeutisch annehmbares Säureadditionssalz überführt.

Die Cyclisierung gemäss Verfahrensvariante a) kann nach an sich bekannten Methoden durchgeführt werden. Die Wahl der geeigneten Methode hängt dabei von der Natur der zu cyclisierenden Verbindung ab. Eine Methode besteht beispielsweise darin, dass man eine entsprechende Verbindung der Formel II auf Temperaturen bis etwa 150 °C erhitzt, wobei in diesem Fall die Anwesenheit eines Lösungsmittels nicht unbedingt erforderlich ist. Andere der hier angesprochenen Verbindungen der Formel II können durch Anwendung von Säure in einem Lösungsmittel oder Lösungsmittelgemisch bei Temperaturen von etwa Raumtemperatur bis etwa 150 °C cyclisiert werden. Eine besonders bevorzugte Säure ist die Essigsäure, welche gleichzeitig auch als Lösungsmittel dienen kann. In einigen Fällen erweist es sich auch als zweckmässig die Verbindungen der Formel II, obwohl an sich isolierbar, ohne Isolierung aus dem Reaktionsgemisch, in dem sie hergestellt worden sind, direkt zu cyclisieren bzw. cyclisieren zu lassen.

Bei der Umzetzung einer Verbindung der Formel II, worin Q die Gruppe (d) bedeutet, mit Cyclopropylnitril bzw. einer Verbindung der Formel II, worin Q die Gruppe (e) bedeutet mit Cyclopropylnitriloxid gemäss Verfahrensvariante b) handelt es sich um eine an sich bekannte Cycloaddition. Geeignete Lösungsmittel für diesen Verfahrensaspekt sind beispielsweise Diäthyläther und Toluol. Die Reaktionstemperatur liegt zweckmässigerweise in einem Bereich von 20 °C bis 80 °C.

Die Herstellung von Verbindungen der Formel I gemäss Verfahrensvariante c) erfolgt ebenfalls nach an sich bekannten Methoden. Die in Formel V mit X" bezeichnete Abgangsgruppe ist vorzugsweise eine leicht abspaltbare Phosphinylgruppe, beispielsweise eine Gruppe der Formel $(R^{10}O)_2POO$-, worin $R^{10}$ niederes Alkyl oder Phenyl bedeutet. Als Basen eignen sich z.B. Alkalimetallalkoxide, wie Kalium-tert.-butoxid, und Alkalimetallhydride, wie Natriumhydrid. Geeignete Lösungsmittel sind beispielsweise Dimethylformamid, Dimethylsulfoxid, Tetrahydrofuran und dergleichen. Die Reaktionstemperatur liegt zweckmässigerweise zwischen etwa -40 °C und etwa Raumtemperatur.

Gemäss Verfahrensvariante d) kann man eine Verbindung der Formel I in ein pharmazeutisch annehmbares Säureadditionssalz überführen. Die Herstellung von solchen pharmazeutisch annehmbaren Säureadditionssalzen erfolgt nach allgemein üblichen Methoden. Es kommen dabei sowohl Salze mit anorganischen als auch Salze mit organischer Säure in Betracht, beispielsweise Hydrochloride, Hydrobromide, Sulfate, Methansulfonate, p-Toluolsulfonate, Oxalate oder, Maleinate.

Die Verbindungen der Formel II, worin Q eine der Gruppen (a) - (d) bedeutet, sind neu und können nach an sich bekannten Methoden wie folgt hergestellt werden:

Eine Verbindung der Formel II, worin Q die Gruppe (a) bedeutet, kann beispielsweise hergestellt werden, indem man eine Verbindung der Formel IIa, worin $Q^1$ die Gruppe $-CONH_2$ bedeutet, mit einer Verbindung der allgemeinen Formel

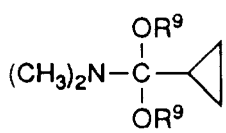 VI

worin $R^9$ niederes Alkyl bedeutet,
umsetzt und das erhaltene Produkt, eine Verbindung der Formel II, worin Q die Gruppe

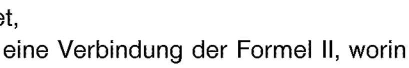

bedeutet mit Hydroxylamin behandelt.

Eine Verbindung der Formel II, worin Q die Gruppe (b) bedeutet, kann beispielsweise hergestellt werden, indem man eine Verbindung der Formel IIa, worin $Q^1$ die Gruppe -COX, und X eine Abgangsgruppe, beispielsweise ein Halogenatom oder eine l-Imidazolylgruppe bedeuten, mit einer Verbindung der Formel

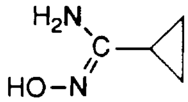

VII

umsetzt.

Eine Verbindung der Formel II, worin Q die Gruppe (c) bedeutet, kann hergestellt werden, indem man beispielsweise eine Verbindung der Formel IIa, worin Q[1] die Gruppe -CN bedeutet, mit Hydroxylamin behandelt und die so erhaltene Verbindung der Formel II, worin Q die Gruppe $-C(NH_2)=NOH$ bedeutet, mit einem reaktiven Derivat einer Carbonsäure der Formel

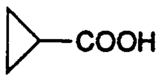

VIIIa

beispielsweise mit einem entsprechenden Anhydrid oder Säurechlorid umsetzt.

Eine Verbindung der Formel II, worin Q die Gruppe (d) bedeutet, kann man dadurch herstellen, dass man in einer Verbindung der Formel IIa, worin Q[1] die Gruppe $-CH=NOH$ bedeutet, die Oximgruppe halogeniert und dann dehydrohalogeniert.

Wie bereits weiter oben erwähnt, ist es nicht notwendig (und in gewissen Fällen auch nicht möglich), die Verbindungen der Formel II, worin Q eine der Gruppen (a)-(c) bedeutet zu isolieren; es erweist sich oft als zweckmässig, diese Verbindungen ohne Isolierung aus dem Reaktionsgemisch, indem sie hergestellt worden sind, direkt zu cyclisieren bzw. cyclisieren zu lassen.

Die Verbindungen der Formel II, worin Q eine der obigen Gruppen (a) - (c) bedeutet, sind neu und ebenfalls Gegenstand der vorliegenden Erfindung.

Die Verbindungen der Formel IIa sind wie bereits erwähnt, an sich bekannt. Die nicht vorbeschriebenen Verbindungen der Formel IIa können in Analogie zu den bekannten Vertretern dieser Stoffklasse hergestellt werden.

Die als Ausgangsstoffe verwendeten Verbindungen der Formeln V und XX sind bekannt oder können in Analogie zu den bekannten Vertretern dieser Stoffklassen hergestellt werden.

Wie eingangs erwähnt sind die Verbindungen der Formel I neu; sie besitzen äusserst wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben ausgeprägte anxiolytische, antikonvulsive, muskelrelaxierende und sedativ-hypnotische Eigenschaften.

Die Affinität von Verbindungen der allgemeinen Formel I zu den zentralen Benzodiazepin-Rezeptoren wurde nach der in Life Science 20, 2101-2110(1977) und Science 198, 849-851(1077) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung des tritiierten Diazepams an die spezifischen Benzodiazepin-Rezeptoren im cerebralen Cortex bewirkt.

Die zentralen Eigenschaften der erfindungsgemässen Verbindungen der Formel I können beispielsweise im nachfolgend beschriebenen und für die Erfassung antikonvulsiver Eigenschaften allgemein anerkannten Antipentetrazoltest nachgewiesen werden.

In diesem Tierversuch verabreicht man 60-80 g schweren, weiblichen Ratten die zu testende Verbindung oral und 30 Minuten später 120 mg/kg i.p. Pentetrazol, das in ungeschützten Versuchstieren 1 bis 4 Minuten nach der Injektion Emprosthotonus und tonische Streckungen der vorderen und/oder hinteren Gliedmassen bewirkt. Man verwendet zehn Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt.

In der nachfolgenden Tabelle werden die Resultate dargestellt, welche mit repräsentativen Vertretern der durch die allgemeine Formel I definierten Verbindungsklasse in den vorstehend geschilderten Versuchen erhalten worden sind. Ausserdem enthält die Tabelle Angaben über die akute Toxizität einiger dieser Verbindungen ($DL_{50}$ in mg/kg bei einmaliger oraler Verabreichung an Mäusen).

| Verbindung der Formel I, worin $R^1$ | $R^2$ | $R^3$ | $R^4$ | $R^5$ | Konfiguration | $IC_{50}$ in nM/l | Antipentetrazol-Test, $ED_{50}$ in mg/kg p.o. | Toxizitäl $DL_{50}$ in mg/kg p.o. |
|---|---|---|---|---|---|---|---|---|
| 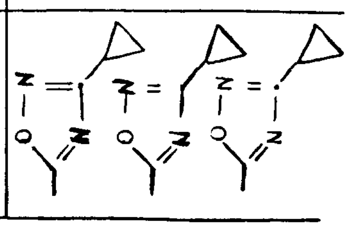 | H | $CH_3$ | Cl | H | - | 1,5 | 0,034 | |
| | $CH_2CH_2CH_2$- | | Cl | H | (S) | 2,2 | 0,20 | 1250-2500 |
| | H | $-CH_3$ | H | F | - | 5,0 | 0,23 | |

Die Verbindungen der Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragees, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragees und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole.

Die pharmazeutischen Präparate können daneben noch Konservierungsmittel, Lösungsvermittler, Stabilisierungsmittel, Netzimittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch weitvolle Stoffe enthalten.

Wie eingangs erwähnt sind Arzneimittel, enthaltend eine Verbindung der Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe in eine galenische Darreichungsform bringt.

Wie eingangs erwähnt, können die Verbindungen der Formel I und ihre pharmazeutisch annehmbaren Säureadditionssalze bei der Bekämpfung bzw. Verhütung von Krankheiten verwendet werden und zwar insbesondere bei der Bekämpfung von Konvulsionen und Angstzuständen. Die Dosierung kann innerhalb weiter Grenzen variieren und ist natürlich in jedem einzelnen Fall den individuellen Gegebenheiten anzupassen. Im allgemeinen kommt bei oraler Verabreichung eine Tagesdosis von 0,01 mg bis 100 mg in Frage.

Die nachfolgenden Beispiele sollen die vorliegende Erfindung näher erläutern. Sämtliche Temperaturen sind in Celsiusgraden angegeben.

## Beispiel 1

a) Eine Mischung aus 37,3 g (116,6 mMol) Aethyl 7-chlor-5,6-dihydro-5-methyl-6-oxo-4H-imidazo[l,5-a]-[1,4]benzodiazepin-3-carboxylat, 5,276 g (131,9 mMol) Natriumhydroxid, 150 ml Aethanol und 100 ml Wasser wird während 15 Minuten unter Rückfluss zum Sieden erhitzt. Dann neutralisiert man durch Zugabe von 132 ml 1N-Salzsäure, destilliert das Aethanol im Vakuum ab und verdünnt mit 100 ml Wasser. Man kühlt auf ca. 0° ab, nutscht ab, wäscht mit Wasser nach und trocknet im Hochvakuum bei 80°. Man erhält 7-Chlor-5,6-dihydro-5-methyl-6-oxo-4H-imidazo[l,5-a][1,4]benzodiazepin-3-carbonsäure vom Zersetzungspunkt 283-284°.

b) 7,3 g (25 mMol) 7-Chlor-5,6-dihydro-5-methyl-6-oxo-4H-imadazo[l,5-a]-[1,4]benzodiazepin-3-carbonsäure werden in 50 ml N,N-Dimethylformamid suspendiert, worauf man mit 5,67 g (34 mMol) 1,1'-Carbonyldiimidazol versetzt und noch während 0,75 Stunden bei Raumtemperatur und anschliessend während 2 Stunden bei 60° rührt. Dann giesst man auf 100 ml Wasser, nutscht nach 1 Stunde ab, wäscht mit Wasser nach und trocknet im Hochvakuum bei 80°. Man erhält 1-[[7-Chlor-5,6-dihydro-5-methyl-6-oxo-4H-imidazo[l,5-a][1,4]-benzodiazepin-3-yl]carbonyl]imadazol vom Zersetzungspunkt 242-244°.

c) 15 g (43,8 mMol) 1-[[7-Chlor-5,6-dihydro-5-methyl-6-oxo-4H-imidazo[l,5-a][1.4]benzodiazepin-3-yl]-carbonyl]imidazol, 6,60 g (65,8 mMol) Cyclopropancarboxamidoxim und 100 ml N,N-Dimethylformamid werden während 1,5 Stunden bei 70° gerührt, worauf man zur Trockene eindampft. Dein Rückstand werden 100 ml Eisessig zugegeben, und die Lösung wird während 1,5 Stunden auf 120° erwärmt. Man dampft dann ein und verteilt den Rückstand zwischen Methylenchlorid und gesättigter Natriumbicarbonat-Lösung. Die organische Phase wird abgetrennt, über Magnesiumsulfat getrocknet und eingedampft. Durch Umkristallisieren des Rückstandes aus Methylenchlorid und Essigester erhält man 7-Chlor-3-(3-cyclopropyl-1,2,4-oxadiazol-5-yl)-5,6-dihydro-5-methyl-4H-imidazo[1,5-a][l,4]benzodiazepin-6-on vom Schmelzpunkt 183-184°.

Beispiel 2

a) 14,5 g (47,7 mMol) (S)-8-Chlor-12,12a-dihydro-9-oxo-9H,11H-azeto[2,1-c]imidazo[1,5-a][1,4]-benzodiazepin-1-carbonsäure werden in 55 ml N,N-Dimethylformamid suspendiert, worauf man portionenweise mit 10,3 g (63,5 mMol) 1,1'-Carbonyldiimidazol versetzt und während 1 Stunde bei Raumtemperatur und während 1,5 Stunden bei 50° rührt. Dann giesst man auf 250 ml Wasser und extrahiert viermal mit Methylenchlorid. Die vereinigten organischen Auszüge werden dreimal mit Wasser gewaschen, über Magnesiumsulfat getrocknet und eingedampft. Zur Reinigung kristallisiert man den Rückstand mittels Essigester. Man erhält l-[[(S)8-Chlor-12,12a-dihydro-9-oxo-9H,llH-azeto[2,1-c]imidazo[1,5-a]-[l,4]benzodiazepin-l-yl]carbonyl]imidazol vom Zersetzungspunkt 223-225°.
b) 15,0 g (42 mMol) l-[[(S)-8-Chlor-12,12a-dihydro-9-oxo-9H,11H-azeto[2,1-c]imidazo[1,5-a][1,4]-benzodiazepin-1-yl]carbonyl]imidazol werden in 60 ml N,N-Dimethylformamid gelöst, worauf man mit 4,60 g (46 mMol) Cyclopropancarboxamidoxim versetzt und während 2 Stunden auf 100° enwärmt. Man dampft zur Trockene ein, versetzt den Rückstand mit 50 ml Essigsäure und rührt während 2 Stunden bei 115°. Nach Eindampfen des Lösungsmittels wird der Rückstand zwischen Methylenchlorid und gesättigter Natriumbicarbonat-Lösung verteilt. Die organische Phase wird abgetrennt, über Magnesiumsulfat getrocknet und eingeengt. Der Rückstand wird unter Eluieren mit Essigester an Kieselgel chromatographiert. Nach Umkristallisieren aus Essigester erhält man (S)-8-Chlor-1-(3-cyclopropyl-1,2,4-oxadiazol-5-yl)-12,12a-dihydro-9H,11H-azeto[2,1-c]-imidazo[1,5-a][1,4]benzodiazepin-9-on vom Schmelzpunkt 140-142°.

Beispiel 3

8,10 g (22 mMol) 1-[[S)-8-Chlor-11,12,13,13a-tetrahydro-9-oxo-9H-imidazo[1,5-a]pyrrolo[2,1-c][1,4]-benzodiazepin-1-yl]carbonyl]imidazol werden in 30 ml N,N-Dimethylformamid gelöst, worauf man mit 2,50 g (25 mMol) Cyclopropancarboxamidoxim versetzt und während 4,5 Stunden auf 70° erwärmt. Dann giesst man das Reaktionsgemisch auf 350 ml Wasser, rührt 20 Minuten und nutscht das ausgefallene Produkt ab. Der Rückstand wird mit Wasser gewaschen und getrocknet. Man löst den obigen Rückstand in 30 ml Eisessig und rührt während 2,5 Stunden bei 115°. Anschliessend dampft man das Reaktionsgemisch ein und chromatographiert den Rückstand unter Eluieren mit Essigester an Kieselgel. Nach Eindampfen der Eluate erhält man (S)-8-Chloro-1-(3-cyclopropyl-1,2,4-oxadiazol-5-yl)-11,12,13,13a-tetrahydro-9H-imidazo-[1,5-a]pyrrolo[2,1-c][1,4]benzodiazepin-9-on vom Schmelzpunkt 213-214°.

Beispiel 4

6,5 g (20 mMol) 1-[(8-Fluor-5,6-dihydro-5-methyl-6-oxo-4H-imidazo[1,5-a][1,4]benzodiazepin-3-yl)-carbonyl]imidazol werden zusammen mit 2,2 g (22 mMol) Cyclopropancarboxamidoxim in 25 ml N,N-Dimethylformamid während 1 Stunde bei 100° gerührt. Dann dampft man zur Trockene ein, gibt zum Rückstand 50 ml Eisessig und erhitzt während 1 Stunde zum Rückfluss. Man dampft wieder im Vakuum zur Trockene ein und löst den Rückstand in Methylenchlorid. Die Methylenchloridlösung wird zweimal mit Natriumbicarbonat-Lösung gewaschen, anschliessend mit Magnesiumsulfat getrocknet und eingedampft. Durch Umkristallisieren aus Methylenchlorid/Essigester erhält man 3-(3-Cyclopropyl-1,2,4-oxadiazol-5-yl)-8-fluor-5,6-dihydro-5-methyl-4H-imidazo[1,5-a][1,4]benzodiazepin-6-on vom Schmelzpunkt 216-217°.

Beispiel 5

a) Eine Mischung aus 6,32 g (20 mMol) (S)-8-Chlor-11,12,13 ,13a-tetrahydro-9-oxo-9H-imidazo[1,5-a]-pyrrolo[2,1-c][l,4]benzodiazepin-1-carboxamid, 5,68 g (14 mMol) 2,4-Bis-(p-methoxyphenyl)-1,3,2,4-dithaidiphosphetan-2,4-disulfid und 100 ml Toluol wird während 4,5 Stunden unter Rückfluss zum Sieden erhitzt. Dann dampft man die erhaltene Lösung im Vakuum ein und chromatographiert den Rückstand unter Eluieren mit Essigester an Kieselgel. Nach Umkrlstallisieren aus Methylenchlorid/Essigester erhält man (S)-8-Chlor-11,12,13,13a-tetrahydro-9-oxo-9H-imidazo[1,5-a]pyrrolo[2,1-c][1,4]benzodiazepin-1-carbonitril vom Schmelzpunkt 237-238°.
b) 70 g (23,4 mMol) (S)-8-Chlor-11,12,13,13a-tetrahydro-9-oxo-9H-imidazo[l,5-a]pyrrolo[2,1-c][1,4]-benzodiazepin-1-carbonitril werden zusammen mit ca. 1,0 g frisch freigesetztem Hydroxylamin in 90 ml Aethanol während 1,5 Stunden bei Siedetemperatur gerührt. Durch Abkühlen, Abfiltrieren des ausgefallenen Materials, Waschen mit Aether und Trocknen erhält man (S)-8-Chloro-11,12,13,13a-tetrahydro-9-oxo-9H-imidazo[1,5-a]pyrrolo[2,1-c][1,4]benzodiazepin-l-carboxamidoxim vom Schmelzpunkt 249-250°. Durch

Chromatographieren des Rückstandes aus der Mutterlauge an Kieselgel und anschliessendes Kristallisieren aus Essigester erhält man noch eine zweite Portion des gewünschten Amidoxims.

c) Zu einer Lösung von 8,61 g (100 mMol) Cyclopropancarbonsäure in 30 ml N,N-Dimethylformamid gibt man portionenweise 17,0 g (105 mMol) N,N'-Carbonyldiimidazol. Man rührt während 1 Stunde bei Raumtempratur und erhitzt dann rasch auf 50°. Man gibt die erhaltene Lösung zu einer Suspension von 33,1g (100 mMol) (S)-8-Chlor-11,12,13,13a-tetrahydro-9-oxo-9H-imidazo[1,5-a]pyrrolo[2,1-c][1,4]-benzodiazpin-1-carboxamidoxim in 100 ml N,N-Dimethylformamid und erwärmt während 2,5 Stunden auf 90°. Man entfernt das Lösungsmittel am Rotationsverdampfer und ersetzt es durch 100 ml Essigsäure. Nach 2,5-stündigem Rühren bei 120° dampft man wieder ein, löst den Rückstand in Methylenchlorid und wäscht ihn mit gesättigter Natriumbicarbonat-Lösung. Man trocknet über Magnesiumsulfat und engt ein. Durch Chromatographieren des öligen Rückstandes an Kieselgel unter Eluieren mit Essigester und Umkristallisieren aus Alkohol und wenig Methylenchlorid erhält man (S)-8-Chlor-1-[5-(cyclopropyl)-1,2,4-oxadiazol-3-yl]-11,12,13,13a-tetrahydro-9H-imidazo[1,5-a]pyrrolo[2,1-c]-[1,4]benzodiazepin-9-on vom Schmelzpunkt 236-237°.

Beispiel 6

Eine Mischung von 9,77 g (39 mMol) 5,6-Dihydro-5-methyl-6-oxo-4H-imidazo[1,5-a][1,4] benzodiazepin-3-carbonsäure, 40 ml N,N-Dimethylformamid und 6,48 g (40 mMol) N,N'-Carbonyldiimidazol wird während 1 Stunde bei 100° gerührt. Man gibt anschliessend 4.01 g (40 mMol) Cyclopropancarbonsäureamidoxim zu und rührt weitere 4 Stunden bei 100°. Man dampft dann ein und erhitzt den Rückstand während 4 Stunden mit 100 ml Essigsäure auf 115°. Die Lösung wird zur Trockene eingedampft, und der Rückstand wird zwischen Methylenchlorid und gesättigter Natriumbicarbonat-Lösung verteilt. Die organische Phase wird abgetrennt, über Magnesiumsulfat getrocknet und eingedampft. Durch Umkristallisieren aus Methylenchlorid und Essigester erhält man 3-(3-Cyclopropyl-1,2,4-oxadiazol-5-yl)-4,5-dihydro-5-methyl-6H-imidazo[l,5-a]-[1,4]-benzodiazepin-6-on vom Schmelzpunkt 202-203°.

Beispiel 7

a) 7,10 g (19 mMol) Aethyl 7-brom-5,6-dihydro-5-methyl-6-oxo-4H-imidazo[1,5-a][1,4] benzodiazepin-3-carboxylat in 30 ml Aethanol werden mit einer Lösung von 1,13 g (28 mMol) Natriumhydroxid in 15 ml Wasser während 20 Minuten zum Sieden erhitzt. Die Lösung wird danach mit 7 ml 4N-Salzsäure neutralisiert und das Aethanol am Rotationsverdampfer entfernt. Man kühlt ab und nutscht den ausgefallenen Festkörper ab. Nach Trocknen im Vakuum bei 85° erhält man 7-Brom-5,6-dihydro-5-methyl-6-oxo -4H-imidazo[1,5-a][1,4]benzodiazepin-3-carbonsäure.

b) 6,2 g (18 mMol) 7-Brom-5,6-dihydro-5-methyl-6-oxo-4H-imidazo[1,5-a]-[1,4]benzodiazepin-3-carbonsäure werden in 90 ml N,N-Dimethylformamid mit 2.92 g (18 mMol) N,N'-Carbonyldiimidazol während 2,5 Stunden bei 70° gerührt. Anschliessend gibt man 1,80 g (18 mMol) Cyclopropancarboxamidoxim zu und rührt weitere 2 Stunden bei 100°. Man dampft die Lösung ein, versetzt den Rückstand mit 70 ml Essigsäure und rührt während 5 Stunden bei 120°. Das Reaktionsgemisch wird eingedampft und der Rückstand zwischen Methylenchlorid und gesättigter Natriumbicarbonat-Lösung verteilt. Die organische Phase wird über Magnesiumsulfat getrocknet und eingedampft. Durch Chromatographieren an Kieselgel unter Eluieren mit Essigester und Umkristallisieren aus Acetonitril erhält man 7-Brom-4,5-dihydro-3-(3-cyclopropyl-1,2,4-oxadiazol-5-yl)-5-methyl-6H-imidazo[1,5-a][1,4]benzodiazepin-6-on vom Schmelzpunkt 187-189°.

Beispiel 8

7,70 g (27,2 mMol) (S)-11,12,13,13a-Tetrahydro-9-oxo-9H-imidazo[1,5-a]-pyrrolo[2,1-c][1,4]-benzodiazepin-l-carbonsäure werden in 40 ml N,N-Dimethylformamid mit 4,41 g (27,2 mMol) N,N'-Carbonyldiimidazol während 10 Minuten bei Raumtemperatur und 20 Minuten bei 85° gerührt. Anschliessend gibt man 2,72 g (27.2 mMol) Cyclopropancarboxamidoxim zu und rührt eine weitere Stunde bei 110°. Nach Entfernen des N,N-Dimethylformamids wird der Rückstand in 30 ml Essigsäure während 4 Stunden bei 120° gerührt. Man dampft die Lösung ein und verteilt den Rückstand zwischen Methylenchlorid und gesättigter Natriumbicarbonat-Lösung. Die organische Phase wird abgetrennt, über Magnesiumsulfat getrocknet und eingedampft. Durch Umkristallisieren aus Essigester erhält man (S)-1-(3-Cyclopropyl-1,2,4-oxadiazol-5-yl)-11,12,13,13a-tetrahydro-9H-imidazo[1,5-a]pyrrolo[2,1-c][1,4]benzodiazepin-9-on vom Schmelzpunkt 211-212°.

Beispiel 9

2,20 g (7 mMol) (R,S)-8-Chlor-11,13a-dihydro-9-oxo-9H-imidazo[1,5-a]pyrrolo[2,1-c][1,4]benzodiazepin-l-carbonsäure, 30 ml N,N-Dimethylformamid und 1,14 g (7 mMol) N,N'-Carbonyldiimidazol werden während 10 Minuten bei Raumtemperatur und 1 Stunde bei 65° gerührt. Anschliessend gibt man 0,70 g (7 mMol) Cyclopropancarbonsäureamidoxim zu und rührt weitere 2 Stunden bei 80°. Man dampft die Lösung ein und versetzt den Rückstand mit 25 ml Essigsäure. Nach 3-stündigem Rühren bei 120° dampft man das Reaktionsgemisch zur Trockene ein und verteilt den Rückstand zwischen Methylenchlorid und gesättigter Natriumbicarbonat-Lösung. Die organische Phase wird abgetrennt und über Magnesiumsulfat getrocknet. Durch Umkristallisieren aus Essigester erhält man (R,S)-8-Chlor-1-(3-cyclopropyl-1,2,4-oxadiazol-5-yl)-11,13a-dihydro-9H-imidazo[1,5-a]pyrrolo[2,1-c][1,4] benzodiazepin-9-on vom Schmelzpunkt 250-252°.

Beispiel 10

Eine Mischung von 11,0 g (30 mMol) 1-[[(S)-8-Chlor-11,12,13,13a-tetrahydro-9-oxo-9H-imidazo[1,5-a]-pyrrolo[2,1-c][l,4]benzodiazepin-l-yl]carbonyl]imidazol, 50 ml N,N-Dimethylformamid und 4,35 g (34 mMol) Cyclopentancarbonsäureamidoxim wird während 3 Stunden bei 85° gerührt. Man dampft dann das Lösungsmittel ab und erhitzt den Rückstand mit 50 ml Essigsäure während 1,5 Stunden auf 115°. Man dampft ein und verteilt den Rückstand zwischen Methylenchlorid und gesättigter Natriumbicarbonat-Lösung. Die organische Phase wird abgetrennt, über Magnesiumsulfat getrocknet und eingedampft. Durch Umkristallisieren aus Essigester und Hexan erhält man (S)-8-Chlor-1-(3-cyclopentyl-l,2,4-oxadiazol-5-yl )-11,12 ,13 ,13a-tetrahydro-9H-imidazo[l, 5-a]pyrrolo[2,1-c][1,4]benzodiazepin-9-on vom Schmelzpunkt 177-178°.

Beispiel 11

a ) 6, 4 g 3, 4-Dihydro-4-methyl-2H-thieno[3, 2-e[1,4]diazepin-2,5(lH)-dion werden in 640 ml Chloroform suspendiert. Man gibt nacheinander 47,6 ml N,N-Dimethyl-p-toluidin und 9,45 ml Phosphoroxychlorid zu und erhitzt unter Rühren 15 Stunden auf Rückflusstemperatur. Das Reaktionsgemisch wird dann abgekühlt und mit 1 l gesättigter wässeriger Natriumhydrogencarbonat-Lösung 30 Minuten intensiv gerührt. Die organische Phase wird abgetrennt und die wässerige Phase noch zweimal mit Chloroform extrahiert. Die Chloroform-Extrakte werden mit Natriumsulfat getrocknet und das Lösungsmittel im Vakuum abdestilliert. Der Rückstand enthält rohes 2-Chlor-3,4-dihydro-4-methyl-2H-thieno[3,2-e][1,4]-diazepin-5(5H)-on, gemischt mit N,N-Dimethyl-p-toluidin.

Durch Zutropfen einer Lösung von 9,2 g Isocyanessigsäure-tert.butylester in 20 ml Dimethylformamid zu einer Lösung von 7,24 g Kaliumtert.-butoxid in 250 ml Dimethylformamid bei -10° bis -5° wird eine Lösung von Isocyanessigsäure-tert.-butylester-Kaliumsalz zubereitet. Dieser Lösung wird bei -10° das oben beschriebene Gemisch von rohem 2-Chlor-3,4-dihydro-4-methyl-2H-thieno[3,2-e][1,4] diazepin-5-(5H)-on und N,N-Dimethyl-p-toluidin zugetropft. Es wird noch 30 Minuten bei -5° und 2 Stunden bei Raumtemperatur weitergerührt. Das Reaktionsgemisch wird dann bei 0° mit 4,9 ml Eisessig versetzt und in 5 l gesättigte wässerige Natriumhydrogencarbonat-Lösung gegossen. Man extrahiert dreimal mit Essigester und zweimal mit Chloroform. Die organischen Phasen werden mit gesättigter Natriumchlorid-Lösung gewaschen und über Natriumsulfat getrocknet. Die Lösungsmittel und der Hauptteil des N,N-Dimethyl-p-toluidins werden im Hochvakuum abdestilliert. Der Rückstand wird durch Silicagel chromatographiert. Mit einem Gemisch von Chloroform und Aethanol (98,8:1,2) wird tert.-Butyl 5,6-dihydro-5-methyl-6-oxo-4H-imidazo[1,5-a]thieno[2,3-f][1,4]diazepin-3-carboxylat eluiert. Der Rückstand aus dem Eluat wird aus Essigsäureäthylester und Diisopropyläther kristallisiert. Man erhält farblose Kristalle von t-Butyl 5,6-dihydro-5-methyl-6-oxo-4H-imidazo[1,5-a]thieno[2,3-f][1,4]diazepin-3-carboxylat vom Schmelzpunkt 178-179°.

b) 7.2 g tert.Butyl 5,6-dihydro-5-methyl-6-oxo-4H-imidazo[1,5-a]thieno[2,3-f][1,4]diazepin-3-carboxylat werden unter Eiskühlung in 100 ml Trifluoressigsäure gelöst und 90 Minuten bei Raumtemperatur stehen gelassen. Die Lösung wird sodann im Vakuum eingedampft und der Rückstand aus Essigsäureäthylester und Diäthyläther kristallisiert. Man erhält 6,65 g rohe 5,6-Dihydro-5-methyl-6-oxo-4H-imidazo[1,5-a]-thieno[2,3-f][1,4]diazepin-3-carbonsäure.

c) 6,65 g Dihydro-5-methyl-6-oxo-4H-imidazo[1,5-a]thieno[2,3-f][1,4]diazepin-3-carbonsäure werden in 200 ml Dimethylformamid suspendiert. Unter Rühren gibt man 4,6 g Carbonyldiimidazol dazu und rührt 75 Minuten bei Raumtemperatur. Man gibt dann 3,0 g Cyclopropancarbonsäureamidoxim zu und erwärmt unter Rühren 13 Stunden auf 80° und 2,5 Stunden auf 120°. Man giesst das Reaktionsgemisch in 4 1 gesättigte wässerige Natriumhydrogencarbonat-Lösung und extrahiert dreimal mit je 800 ml

Essigsäureäthylester. Die organischen Extrakte werden mit gesättigter Natriumchlorid-Lösung gewaschen, über Natriumsulfat getrocknet und im Vakuum eingedampft. Der Rückstand wird durch Silicagel chromatographiert. Mit einem Gemisch von Chloroform und Aethanol (99,4:0,6) wird 3-(3-Cyclopropyl-1,2,4-oxadiazol-5-yl)-5,6-dihydro-5-methyl-4H-imidazo[1,5-a]thieno[2,3-f][1,4]diazepin-6-on eluiert. Dieses wird aus Essigsäureäthylester umkristallisiert, Smp. 204-205°.

Beispiel 12

a) In 1,4 1 Dichlormethan werden nacheinander 44,3 g Cyanessigsäure und 59,2 g Sarcosinäthylester gelöst. Zu dieser Lösung tropft man innerhalb von 15 Minuten, bei 15-20°, eine Lösung von 114g Dicyclohexylcarbodiimid in 1,1 l Dichlormethan und rührt noch 1 Stunde bei Raumtemperatur und anschliessend 30 Minuten bei 5°. Der ausgefallene Dicyclohexylharnstoff wird abfiltriert und das Filtrat wird im Vakuum eingedampft. Der Rückstand wird in Toluol gelöst und durch Kieselgel chromatographiert. Mit Toluol/Essigsäureäthylester 2:1 wird öliger N-(Cyanacetyl)sarcosinäthylester eluiert.

b) Zu einer Suspension von 30,7 g 2,5-Dihydroxy 1,4-dithian in 600 ml Aethanol werden 74,5 g N-(Cyanacetyl)sarcosin-äthylester zugegeben. Die Suspension wird auf 50° erwärmt, worauf ein (Gemisch von 10 ml Piperidin und 20 ml Triäthylamin zugetropft wird. Dann wird das erhaltene Gemisch 2 1/4 Stunden bei 75° gerührt. Eine kleine Menge unlösliches Material wird sodann wegfiltriert, und das Filtrat wird im Vakuum eingedampft. Der Rückstand wird in 2% Aethanol enthaltendem Chloroform gelöst und durch Kieselgel chromatographiert. Mit Chloroform-Aethanol 98:2 wird rohes 3,4-Dihydro-4-methyl-2H-thieno[2,3-e][1,4]diazepin-2,5(lH)-dion eluiert. Nach Aufkochen in Toluol/Chloroform erhält man Kristalle vom Smp. 235-238°. Ein aus Aethanol umkristallisiertes Muster schmilzt bei 238-239°.

c) Aus 8,0 g 3,4-Dihydro-4-methyl-2H-thieno[2,3-e][1,4]diazepin-2,5(lH)-dion erhält man in Analogie zu dem in Beispiel 11a) beschriebenen Verfahren 5,6-Dihydro-5-methyl-4-oxo-4H-imidazo[1,5-a]thieno[3,2-f]-[1,4]diazepin-7-carbonsäure-tert-bulylester vom Smp. 199-200° (umkristallisiert aus Diäthyläther-Diisopropyläther).

d) Aus 6,1 g 5,6-Dihydro-5-methyl-4-oxo-4H-imidazo[l,5-a]thieno[3,2-f][1,4]diazepin-7-carbonsäure-tert-butylester erhält man in Analogie zu dem in Beispiel 11b) beschriebenen Verfahren 5,6-Dihydro-5-methyl-4-oxo-4H-imidazo[1,5-a]thieno[3,2-f][1,4]diazepin-7-carbonsäure vom Smp. (Zers.) 262-263° (umkristallisiert aus Essigsäureäthylester).

e) Aus 4,9 g 5,6-Dihydro-5-methyl-4-oxo-4H-imidazo[1,5-a]thieno[3,2-f][1,4]diazepin-7-carbonsäure erhält man in Analogie zu dem in Beispiel 11c) beschriebenen Verfahren 7-(3-Cyclopropyl-1,2,4-oxadiazol-5-yl)-5,6-dihydro-4H-imidazo[1,5-a]thieno[3,2-f][1,4]diazepin-4-on vom Smp. 194-195° (umkristallisiert aus Essigsäureäthylester/Diisopropyläther).

Beispiel A

Es werden in üblicher Weise Tabletten folgender Zusammensetzung hergestellt:

| | mg/Tablette |
|---|---|
| Wirkstoff | 0,2 |
| Milchzucker | 140 |
| Maisstärke | 50,8 |
| Polyvinylpyrrolidin | 8 |
| Magnesiumstearat | 1 |
| Tablettengewicht | 200 |

Beispiel B

Es werden in üblicher Weise Kapseln folgender Zusammensetzung hergestellt:

| | mg/Kapsel |
|---|---|
| Wirkstoff | 0,5 |
| Milchzucker | 40 |
| Maisstärke | 8 |
| Talk | |
| Magnesiumstearat | 0,5 |
| Kapselfüllgewicht | 50 |

Die nachstehend aufgeführten Verbindungen der Formel I können wie in den obigen Beispielen A und B angegeben als Wirkstoffe verwendet werden:

7-Chlor-3-( 3-cyclopropyl-1,2,4-oxadiazol-5-yl )-5,6-dihydro-5-methyl-4H-imidazo[1,5-a][1,4]-benzodiazepin-6-on,

( S )-8-Chlor-1-( 3-cyclopropyl-1,2,4-oxadiazol-5-yl)-12,12a-dihydro-9H,11H-azeto[2,1-c]imidazo[1,5-a]-[1,4]benzodiazepin-9-on,

(S)-8-Chlor-1-(3-cyclopropyl-1,2,4-oxadiazol-5-yl)-11,12,13,13a-tetrahydro-9H-imidazo[1,5-a]pyrrolo[2,1-c][1,4]benzodiazepin-9-on und

3-(3-Cyclopropyl-1,2,4-oxadiazol-5-yl)-8-fluor-5,6-dihydro-5-methyl-4H-imidazo[1,5-a][1,4]benzodiazepin-6-on.

**Patentansprüche**

**Patentansprüche für folgende Vertragsstaaten : BE, CH, DE, FR, GB, IT, LI, LU, NL, SE**

**1.** Verbindungen der allgemeinen Formel

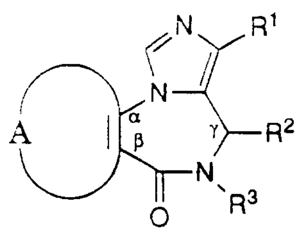

I

worin A zusammen mit den beiden mit $\alpha$ und $\beta$ bezeichneten Kohlenstoffatomen eine der Gruppen

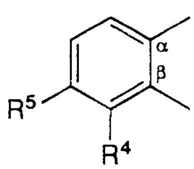

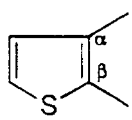

und

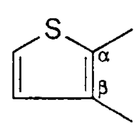

(1) (2) (3)

und worin $R^1$ 3-Cyclopropyl-1,2,4-oxadiazol-5-yl oder 5-Cyclopropyl-1,2,4-oxadiazol-3-yl, $R^2$ Wasserstoff und $R^3$ $C_1$-$C_7$-Alkyl oder $R^2$ und $R^3$ zusammen Dimethylen, Trimethylen oder Propenylen und $R^4$ und $R^5$ je Wasserstoff oder Halogen bedeuten, wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen, Trimethylen oder Propenylen bedeuten,
und pharmazeutisch annehmbare Säureadditionssalze davon.

**2.** Verbindungen gemäss Anspruch 1, dadurch gekennzeichnet, dass A die Gruppe (1) bedeutet.

**3.** Verbindungen gemäss Anspruch 1 oder 2, dadurch gekennzeichnet, dass $R^2$ Wasserstoff und $R^3$ $C_1$-$C_7$-Alkyl oder $R^2$ und $R^3$ zusammen Dimethylen oder Trimethylen bedeuten und dass sie bezüglich des mit $\gamma$ bezeichneten Kohlenstoffatoms die (S)-Konfiguration aufweisen, wenn $R^2$ und $R^3$ zusammen Dimethylen oder Trimethylen bedeuten.

**4.** 7-Chlor-3-(3-cyclopropyl-1,2,4-oxadiazol-5-yl)-5,6-dihydro-5-methyl-4H-imidazo[1,5-a][1,4]-benzodiazepin-6-on.

**5.** (S)-8-Chlor-1-(3-cyclopropyl-1,2,4-oxadiazol-5-yl)-12,12a-dihydro-9H,11H-azeto[2,1-c]imidazo[1,5-a][1,4]-benzodiazepin-9-on.

**6.** (S)-8-Chlor-1-(3-cyclopropyl-1,2,4-oxadiazol-5-yl)-11,12,13,13a-tetrahydro-9H-imidazo[ 1,5-a]pyrrolo[2,1-c][1,4]benzodiazepin-9-on.

**7.** 3-(3-Cyclopropyl-1,2,4-oxadiazol-5-yl)-8-fluor-5,6-dihydro-5-methyl-4H-imidazo[1,5-a][1,4]benzodiazepin-6-on.

**8.** Verbindungen der allgemeinen Formel

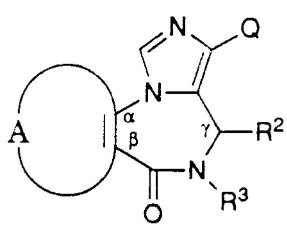

II

worin Q die Gruppe

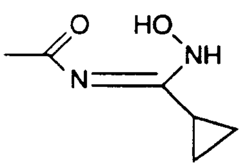

(a) ,

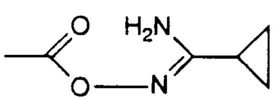

(b) oder

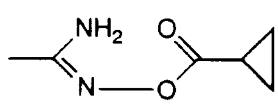

(c)

bedeutet und $R^2$, $R^3$ und A die in Anspruch 1 angegebene Bedeutung besitzen.

**9.** Verbindungen gemäss einem der Ansprüche 1-7 zur Anwendung als therapeutische Wirkstoffe.

**10.** Verbindungen gemäss einem der Ansprüche 1-7 zur Anwendung als antikonvulsive, anxiolytische, muskelrelaxierende und sedativ-hypnotische Wirkstoffe.

**11.** 7-Chlor-3-(3-cyclopropyl-1,2,4-oxadiazol-5-yl)-5,6-dihydro-5-methyl-4H-imidazo[1,5-a][1,4]-benzodiazepin-6-on als Verbindung gemäss Anspruch 9 oder 10.

**12.** Verfahren zur Herstellung von Verbindungen gemäss einem der Ansprüche 1-7 und deren pharmazeutisch annehmbaren Säureadditionssalzen, dadurch gekennzeichnet, dass man ausgehend von Verbindungen der allgemeinen Formel

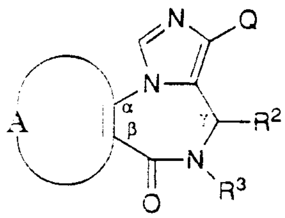

II

worin $R^2$, $R^3$ und A die in Anspruch 1 angegebene und Q die nachstehend angegebene Bedeutung besitzen.

a) eine Verbindung der Formel II, worin Q die Gruppe

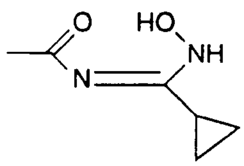 (a) , 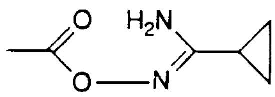 (b) oder 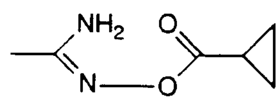 (c)

bedeutet,

cyclisiert, oder

b) eine Verbindung der Formel II, worin Q die Gruppe $-C \equiv N \rightarrow O$ (d) bedeutet, mit Cyclopropylnitril oder eine Verbindung der Formel II, worin Q die Gruppe -CN (e) bedeutet, mit Cyclopropylnitriloxid umsetzt, oder dadurch, dass man

c) eine Verbindung der allgemeinen Formel

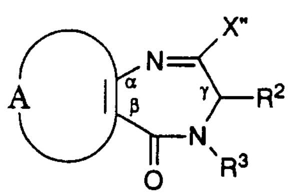

V

worin X" eine Abgangsgruppe bedeutet und $R^2$, $R^3$ und A die in Anspruch 1 angegebene Bedeutung besitzen,

in Gegenwart einer Base mit einem Isonitril der allgemeinen Formel

$CN-CH_2-R^1$ XX

worin $R^1$ die in Anspruch 1 angegebene Bedeutung besitzt,

umsetzt; und dass man

d) erwünschtenfalls eine erhaltene Verbindung der Formel I in ein pharmazeutisch annehmbares Säureadditionssalz überführt.

**13.** Arzneimittel, enthaltend eine Verbindung gemäss einem der Ansprüche 1-7 und ein therapeutisch inertes Excipiens.

**14.** Antikonsulsiv, anxiolytisch, muskelrelaxierend und sedativ-hypnotisch wirksame Arzneimittel gemäss Anspruch 13.

**15.** Arzneimittel gemäss Anspruch 13 oder 14, enthaltend 7-Chlor-3-(3-cyclopropyl-1,2,4-oxadiazol-5-yl)-5,6-dihydro-5-methyl-4H-imidazo[1,5-a]-[1,4]benzodiazepin-6-on.

**Patentansprüche für folgenden Vertragsstaat : AT**

**1.** Verfahren zur Herstellung von Verbindungen der allgemeinen Formel

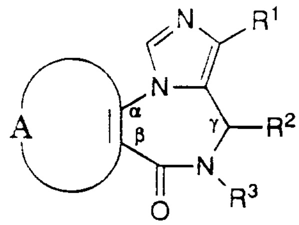

I

worin A zusammen mit den beiden mit $\alpha$ und $\beta$ bezeichneten Kohlenstoffatomen eine der Gruppen

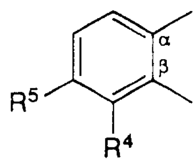

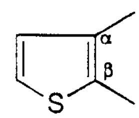

und

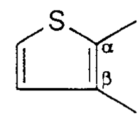

(1) (2) (3)

und worin $R^1$ 3-Cyclopropyl-1,2,4-oxadiazol-5-yl oder 5-Cyclopropyl-1,2,4-oxadiazol-3-yl, $R^2$ Wasserstoff und $R^3$ $C_1$-$C_7$-Alkyl oder $R^2$ und $R^3$ zusammen Dimethylen, Trimethylen oder Propenylen und $R^4$ und $R^5$ je Wasserstoff oder Halogen bedeuten, wobei die Verbindungen der Formel I bezüglich des mit $\gamma$ bezeichneten Kohlenstoffatoms die (S)- oder (R,S)-Konfiguration aufweisen, wenn $R^2$ und $R^3$ zusammen Dimethylen, Trimethylen oder Propenylen bedeuten,
und von pharmazeutisch annehmbaren Säureadditionssalzen davon, dadurch gekennzeichnet, dass man ausgehend von Verbindungen der allgemeinen Formel

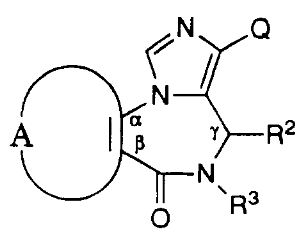

II

worin $R^2$, $R^3$ und A die in Anspruch 1 angegebene und Q die nachstehend angegebene Bedeutung besitzen,

a) eine Verbindung der Formel II, worin Q die Gruppe

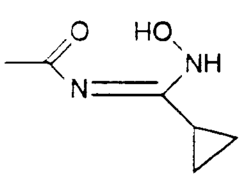

(a) ,

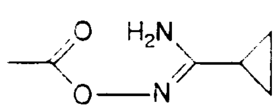

(b) oder

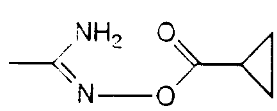

(c)

bedeutet,

cyclisiert, oder

b) eine Verbindung der Formel II, worin Q die Gruppe -C≡N→O (d) bedeutet, mit Cyclopropylnitril oder eine Verbindung der Formel II, worin Q die Gruppe -CN (e) bedeutet, mit Cyclopropylnitriloxid umsetzt, oder dadurch, dass man

c) eine Verbindung der allgemeinen Formel

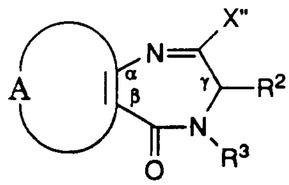

V

worin X" eine Abgangsgruppe bedeutet und $R^2$, $R^3$ und A obige Bedeutung besitzen, in Gegenwart einer Base mit einem Isonitril der allgemeinen Formel

$CN-CH_2-R^1$   XX

worin $R^1$ obige Bedeutung besitzt, umsetzt; und dass man

d) erwünschtenfalls eine erhaltene Verbindung der Formel I in ein pharmazeutisch annehmbares Säureadditionssalz überführt.

**2.** Verfahren gemäss Anspruch 1, dadurch gekennzeichnet, dass A die Gruppe (1) bedeutet.

**3.** Verfahren gemäss Anspruch 1 oder 2, dadurch gekennzeichnet, dass $R^2$ Wasserstoff und $R^3$ $C_1$-$C_7$-Alkyl oder $R^2$ und $R^3$ zusammen Dimethylen oder Trimethylen bedeuten und dass bezüglich des mit $\gamma$ bezeichneten Kohlenstoffatoms die (S)-Konfiguration vorliegt, wenn $R^2$ und $R^3$ zusammen Dimethylen oder Trimethylen bedeuten.

**4.** Verfahren gemäss Anspruch 1, dadurch gekennzeichnet, dass man 7-Chlor-3-(3-cyclopropyl-1,2,4-oxadiazol-5-yl)-5,6-dihydro-5-methyl-4H-imidazo[1,5-a][1,4]benzodiazepin-6-on herstellt.

**5.** Verfahren gemäss Anspruch 1, dadurch gekennzeichnet, dass man (S)-8-Chlor-1-(3-cyclopropyl-1,2,4-oxadiazol-5-yl)-12,12a-dihydro-9H,11H-azeto[2,1-c]imidazo[1,5-a][1,4]benzodiazepin-9-on herstellt.

**6.** Verfahren gemäss Anspruch 1, dadurch gekennzeichnet, dass man (S)-8-Chlor-1-(3-cyclopropyl-1,2,4-oxadiazol-5-yl)-11,12,13,13a-tetrahydro-9H-imidazo[1,5-a]pyrrolo[2,1-c][1,4]benzodiazepin-9-on herstellt.

**7.** Verfahren gemäss Anspruch 1, dadurch gekennzeichnet, dass man 3-(3-Cyclopropyl-1,2,4-oxadiazol-5-yl)-8-fluor-5,6-dihydro-5-methyl-4H-imidazo[1,5-a][1,4]benzodiazepin-6-on herstellt.

## Claims

**Claims for the following Contracting States : BE, CH, DE, FR, GB, IT, LI, LU, NL, SE**

**1.** Compounds of the general formula

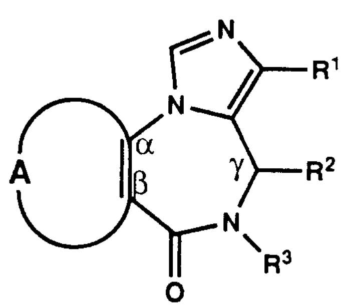

I

wherein A together with the two carbon atoms denoted by $\alpha$ and $\beta$ signifies one of the groups

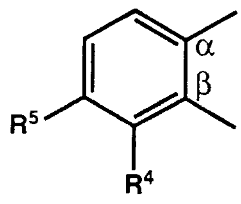

(1)

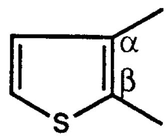

(2)

and

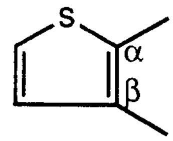

(3)

and wherein $R^1$ signifies 3-cyclopropyl-1,2,4-oxadiazol-5-yl or 5-cyclopropyl-1,2,4-oxadiazol-3-yl, $R^2$ signifies hydrogen and $R^3$ signifies $C_1$-$C_7$-alkyl or $R^2$ and $R^3$ together signify dimethylene, trimethylene or propenylene and $R^4$ and $R^5$ each signify hydrogen or halogen, the compounds of formula I having the (S) or (R,S) configuration with reference to the carbon atom denoted by $\gamma$ when $R^2$ and $R^3$ together signify dimethylene, trimethylene or propenylene,
and pharmaceutically acceptable acid addition salts thereof.

**2.** Compounds in accordance with claim 1, characterized in that A signifies group (1).

**3.** Compounds in accordance with claim 1 or 2, characterized in that $R^2$ signifies hydrogen and $R^3$ signifies $C_1$-$C_7$-alkyl or $R^2$ and $R^3$ together signify dimethylene or trimethylene and wherein they have the (S) configuration with respect to the carbon atom denoted by $\gamma$ when $R^2$ and $R^3$ together signify dimethylene or trimethylene.

**4.** 7-Chloro-3-(3-cyclopropyl-1,2,4-oxadiazol-5-yl)-5,6-dihydro-5-methyl-4H-imidazo[1,5-a][1,4]-benzodiazepin-6-one.

**5.** (S)-8-Chloro-1-(3-cyclopropyl-1,2,4-oxadiazol-5-yl)-12,12a-dihydro-9H,11H-azeto[2,1-c]imidazo[1,5-a]-[1,4]benzodiazepin-9-one.

**6.** (S)-8-Chloro-1-(3-cyclopropyl-1,2,4-oxadiazol-5-yl)-11,12,13,13a-tetrahydro-9H-imidazo[1,5-a]pyrrolo[2,1-c][1,4]benzodiazepin-9-one.

**7.** 3-(3-Cyclopropyl-1,2,4-oxadiazol-5-yl)-8-fluoro-5,6-dihydro-5-methyl-4H-imidazo[1,5-a][1,4]-benzodiazepin-6-one.

**8.** Compounds of the general formula

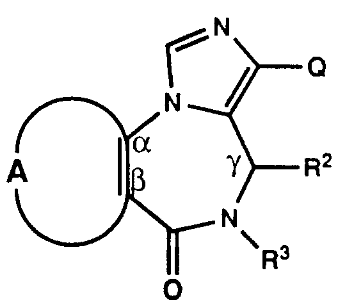

II

wherein Q signifies the group

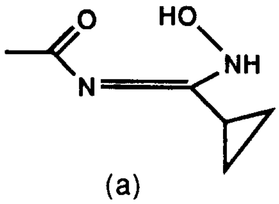
,
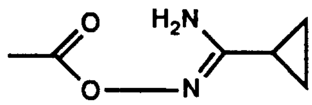
or
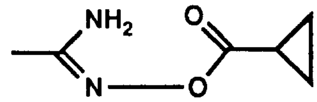

(a) (b) (c)

and $R^2$, $R^3$ and A have the significance given in claim 1.

**9.** Compounds in accordance with any one of claims 1-7 for use as therapeutically active substances.

**10.** Compounds in accordance with any one of claims 1-7 for use as anticonvulsant, anxiolytic, muscle relaxant and sedative-hypnotic active substances.

**11.** 7-Chloro-3-(3-cyclopropyl-1,2,4-oxadiazol-5-yl)-5,6-dihydro-5-methyl-4H-imidazo[1,5-a][1,4]-benzodiazepin-6-one as a compound in accordance with claim 9 or 10.

**12.** A process for the manufacture of compounds of formula I in accordance with any one of claims 1-7 and their pharmaceutically acceptable acid addition salts, characterized by starting from compounds of the general formula

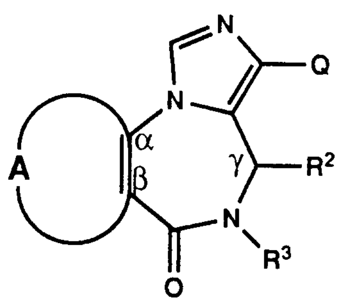
II

wherein $R^2$, $R^3$ and A have the significance given in claim 1 and Q has the significance given hereinafter,
and
a) cyclizing a compound of formula II in which Q signifies the group

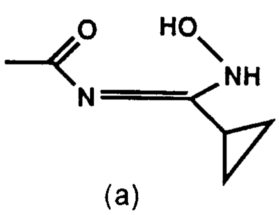
,
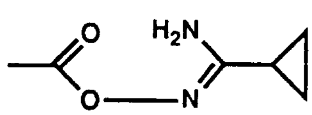
or
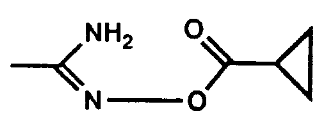

(a) (b) (c)

or
b) reacting a compound of formula II in which Q signifies the group -C≡N→O (d) with cyclopropyl nitrile or reacting a compound of formula II in which Q signifies the group -CN (e) with cyclopropyl nitrile oxide, or

c) reacting a compound of the general formula

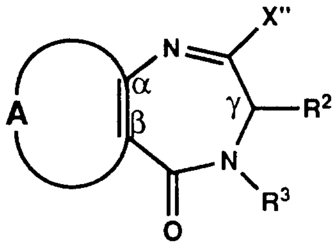

V

wherein X" signifies a leaving group and $R^2$, $R^3$ and A have the significance given in claim 1, in the presence of a base with an isonitrile of the general formula

$CN-CH_2-R^1$ XX

wherein $R^1$ has the significance given in claim 1,
and whereafter
d) if desired, a compound of formula I obtained is converted into a pharmaceutically acceptable acid addition salt.

**13.** A medicament containing a compound in accordance with any one of claims 1-7 and a therapeutically inert excipient.

**14.** A medicament in accordance with claim 13 having anticonvulsant, anxiolytic, muscle relaxant and sedative-hypnotic activity.

**15.** A medicament in accordance with claim 13 or 14, containing 7-chloro-3-(3-cyclopropyl-1,2,4-oxadiazol-5-yl)-5,6-dihydro-5-methyl-4H-imidazo[1,5-a][1,4]benzodiazepin-6-one.

**Claims for the following Contracting State : AT**

**1.** A process for the manufacture of compounds of the general formula

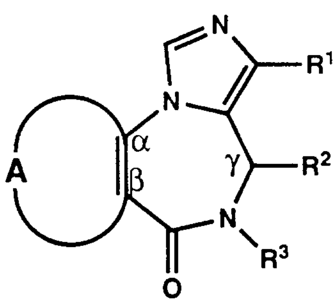

I

wherein A together with the two carbon atoms denoted by α and β signifies one of the groups

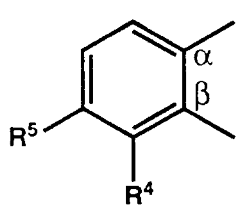

(1)

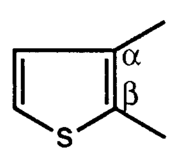

(2)

and

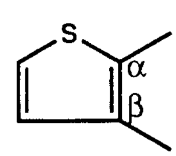

(3)

and wherein $R^1$ signifies 3-cyclopropyl-1,2,4-oxadiazol-5-yl or 5-cyclopropyl-1,2,4-oxadiazol-3-yl, $R^2$ signifies hydrogen and $R^3$ signifies $C_1$-$C_7$-alkyl or $R^2$ and $R^3$ together signify dimethylene, trimethylene or propenylene and $R^4$ and $R^5$ each signify hydrogen or halogen, the compounds of formula I having the (S) or (R,S) configuration with reference to the carbon atom denoted by $\gamma$ when $R^2$ and $R^3$ together signify dimethylene, trimethylene or propenylene,
and pharmaceutically acceptable acid addition salts thereof, characterized by starting from compounds of the general formula

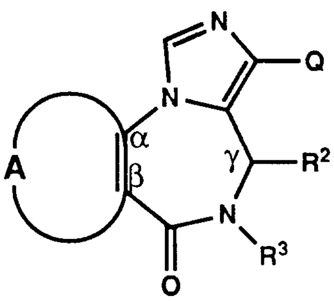

II

wherein $R^2$, $R^3$ and A have the significance given in claim 1 and Q has the significance given hereinafter,
and
a) cyclizing a compound of formula II in which Q signifies the group

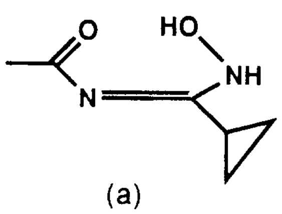

(a)

,

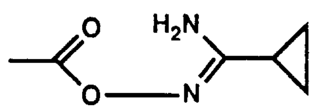

(b)

or

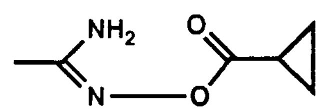

(c)

or
b) reacting a compound of formula II in which Q signifies the group -C≡N→O (d) with cyclopropyl nitrile or reacting a compound of formula II in which Q signifies the group -CN (e) with cyclopropyl nitrile oxide, or
c) reacting a compound of the general formula

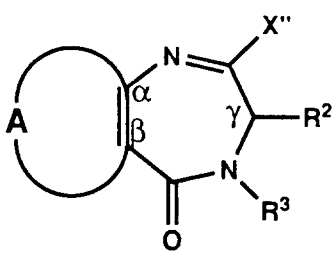

V

wherein X" signifies a leaving group and $R^2$, $R^3$ and A have the above significance,
in the presence of a base with an isonitrile of the general formula

$CN\text{-}CH_2\text{-}R^1$    XX

wherein $R^1$ has the above significance,
and whereafter
d) if desired, a compound of formula I obtained is converted into a pharmaceutically acceptable acid addition salt.

2. A process in accordance with claim 1, characterized in that A signifies group (1).

3. A process in accordance with claim 1 or 2, characterized in that $R^2$ signifies hydrogen and $R^3$ signifies $C_1$-$C_7$-alkyl or $R^2$ and $R^3$ together signify dimethylene or trimethylene and the (S) configuration pertains with respect to the carbon atom denoted by $\gamma$ when $R^2$ and $R^3$ together signify dimethylene or trimethylene.

4. A process according to claim 1, characterized in that 7-chloro-3-(3-cyclopropyl-1,2,4-oxadiazol-5-yl)-5,6-dihydro-5-methyl-4H-imidazo[1,5-a][1,4]benzodiazepin-6-one is manufactured.

5. A process according to claim 1, characterized in that (S)-8-chloro-1-(3-cyclopropyl-1,2,4-oxadiazol-5-yl)-12,12a-dihydro-9H,11H-azeto[2,1-c]imidazo[1,5-a][1,4]benzodiazepin-9-one is manufactured.

6. A process according to claim 1, characterized in that (S)-8-chloro-1-(3-cyclopropyl-1,2,4-oxadiazol-5-yl)-11,12,13, 13a-tetrahydro-9H-imidazo[1,5-a]pyrrolo[2,1-c][1,4]benzodiazepin-9-one is manufactured.

7. A process according to claim 1, characterized in that 3-(3-cyclopropyl-1,2,4-oxadiazol-5-yl)-8-fluoro-5,6-dihydro-5-methyl-4H-imidazo[1,5-a][1,4]benzodiazepin-6-one is manufactured.

## Revendications

**Revendications pour les Etats contractants suivants : BE, CH, DE, FR, GB, IT, LI, LU, NL, SE**

1. Composés de formule générale

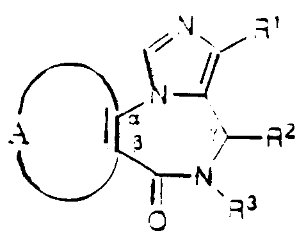

I

dans laquelle A forme avec les deux atomes de carbone $\alpha$ et $\beta$ l'un des groupes

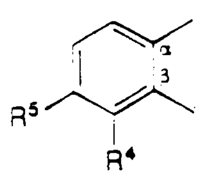

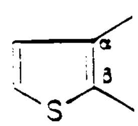

et

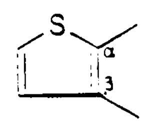

(1) (2) (3)

et dans laquelle $R^1$ représente un groupe 3-cyclopropyl-1,2,4-oxadiazole-5-yle ou 5-cyclopropyl-1,2,4-oxadiazole-3-yle, $R^2$ représente l'hydrogène et $R^3$ un groupe alkyle en C 1-C 7, ou bien $R^2$ et $R^3$ forment ensemble un groupe diméthylène, triméthylène ou propénylène et $R^4$ et $R^5$ représentent chacun l'hydrogène ou un halogène, les composés de formule I étant en configuration (S) ou (R,S) par rapport à l'atome de carbone $\gamma$ lorsque $R^2$ et $R^3$ forment ensemble un groupe diméthylène, triméthylène ou propénylène, et leurs sels formés par addition avec des acides acceptables pour l'usage pharmaceutique.

2. Composés selon revendication 1, caractérisés en ce que A représente le groupe (1).

3. Composés selon revendication 1 ou 2, caractérisés en ce que $R^2$ représente l'hydrogène et $R^3$ un groupe alkyle en C 1-C 7 ou bien $R^2$ et $R^3$ forment ensemble un groupe diméthylène ou triméthylène et en ce qu'ils sont en configuration (S) par rapport à l'atome de carbone $\gamma$ lorsque $R^2$ et $R^3$ forment ensemble un groupe diméthylène ou triméthylène.

4. La 7-chloro-3-(3-cyclopropyl-1,2,4-oxadiazole-5-yl)-5,6-dihydro-5-méthyl-4H-imidazo[1,5-a] [1,4]-benzodiazépine-6-one.

5. La (S)-8-chloro-1-(3-cyclopropyl-1,2,4-oxadiazole-5-yl)-12,12a-dihydro-9H,11H-azéto[2,1-c] imidazo[1,5-a] [1,4]-benzodiazépine-9-one.

6. La (S)-8-chloro-1-(3-cyclopropyl-1,2,4-oxadiazole-5-yl)-11,12,13,13a-tétrahydro-9H-imidazo[1,5-a]-pyrrolo[2,1-c] [1,4]benzodiazépine-9-one.

7. La 3-(3-cyclopropyl-1,2,4-oxadiazole-5-yl)-8-fluoro-5,6-dihydro-5-méthyl-4H-imidazo[1,5-a] [1,4]-benzodiazépine-6-one.

8. Composés de formule générale

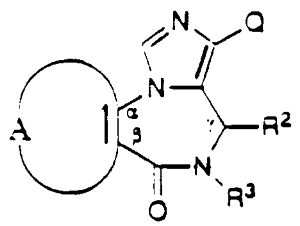

II

dans laquelle Q représente le groupe

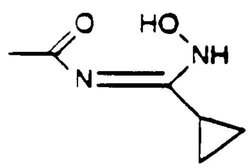
(a) ,
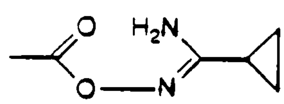
(b) ou
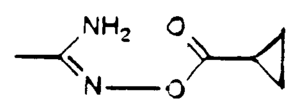
(c)

et $R^2$, $R^3$ et A ont les significations indiquées dans la revendication 1.

9. Composés selon une des revendications 1 à 7, pour l'utilisation en tant que substances actives thérapeutiques.

10. Composés selon une des revendications 1 à 7, pour l'utilisation en tant que substances actives anticonvulsives, anxiolytiques, myorelaxantes et sédatives-hypnotiques.

11. La 7-chloro-3-(3-cyclopropyl-1,2,4-oxadiazole-5-yl)-5,6-dihydro-5-méthyl-4H-imidazo[1,5-a] [1,4]-benzodiazépine-6-one en tant que composé selon revendication 9 ou 10.

12. Procédé de préparation des composés selon une des revendications 1 à 7 et de leurs sels formés par addition avec des acides acceptables pour l'usage pharmaceutique, caractérisé en ce que, partant de composés de formule générale

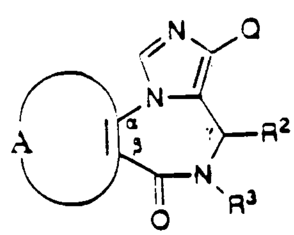

II

dans laquelle $R^2$, $R^3$ et A ont les significations indiquées dans la revendication 1, et Q les significations indiquées ci-après,

a) on cyclise un composé de formule II dans laquelle Q représente le groupe

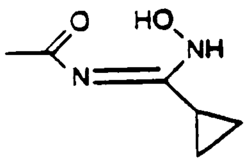 (a) , 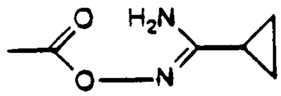 (b) ou 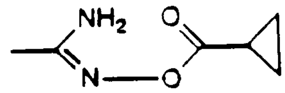 (c)

ou bien

b) on fait réagir un composé de formule II dans laquelle Q représente le groupe -C≡N→O (d), avec le cyclopropylnitrile, ou bien un composé de formule II dans laquelle Q représente le groupe -CN (e) avec l'oxyde du cyclopropylnitrile, ou bien

c) on fait réagir un composé de formule générale

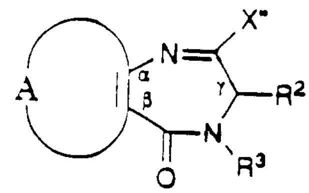

V

dans laquelle X'' représente un groupe éliminable, et $R^2$, $R^3$ et A ont les significations indiquées dans la revendication 1, en présence d'une base, avec un isonitrile de formule générale

$CN\text{-}CH_2\text{-}R^1$ XX

dans laquelle $R^1$ a les significations indiquées dans la revendication 1, et

d) si on le désire, on convertit un composé ainsi obtenu, répondant à la formule I, en un sel formé par addition avec un acide acceptable pour l'usage pharmaceutique.

**13.** Médicament contenant un composé selon une des revendications 1 à 7 et un excipient inerte du point de vue thérapeutique.

**14.** Médicament à activité anticonvulsive, anxiolytique, myorelaxante et sédative-hypnotique selon revendication 13.

**15.** Médicament selon revendication 13 ou 14, contenant de la 7-chloro-3-(3-cyclopropyl-1,2,4-oxadiazole-5-yl)-5,6-dihydro-5-méthyl-4H-imidazo[1,5-a] [1,4]benzodiazépine-6-one.

**Revendications pour l'Etat contractant suivant : AT**

**1.** Procédé de préparation des composés de formule générale

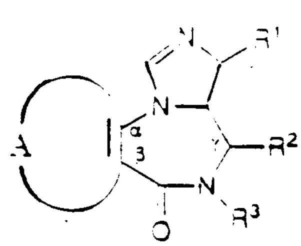

I

dans laquelle A forme avec les deux atomes de carbone $\alpha$ et $\beta$ l'un des groupes

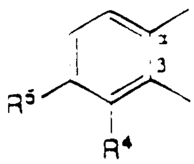
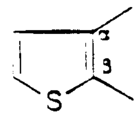
et
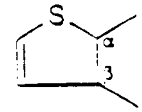

(1) (2) (3)

et $R^1$ représente un groupe 3-cyclopropyl-1,2,4-oxadiazole-5-yle ou 5-cyclopropyl-1,2,4-oxadiazole-3-yle, $R^2$ représente l'hydrogène et $R^3$ un groupe alkyle en C 1-C 7, ou bien $R^2$ et $R^3$ forment ensemble un groupe diméthylène, triméthylène ou propénylène, et $R^4$ et $R^5$ représentent chacun l'hydrogène ou un halogène, les composés de formule I étant en configuration (S) ou (R,S) par rapport à l'atome de carbone $\gamma$ lorsque $R^2$ et $R^3$ forment ensemble un groupe diméthylène, triméthylène ou propénylène, et de leurs sels formés par addition avec des acides acceptables pour l'usage pharmaceutique, caractérisé en ce que, partant de composés de formule générale

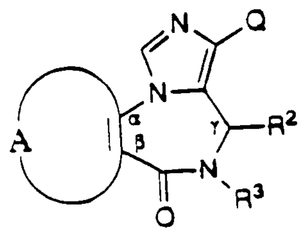
II

dans laquelle $R^2$, $R^3$ et A ont les significations indiquées ci-dessus et Q les significations indiquées ci-après,

a) on cyclise un composé de formule II dans laquelle Q représente le groupe

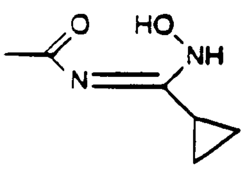
(a) ,
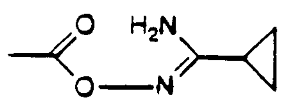
(b) ou
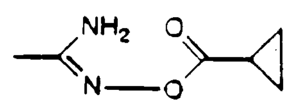
(c)

ou bien

b) on fait réagir un composé de formule II dans laquelle Q représente le groupe -C≡N→O (d) avec le cyclopropylnitrile ou un composé de formule II dans laquelle Q représente le groupe -CN (e) avec l'oxyde du cyclopropylnitrile, ou bien

c) on fait réagir un composé de formule générale

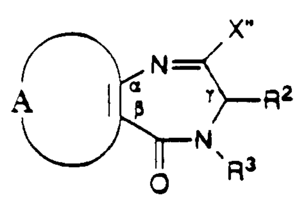
V

dans laquelle X" représente un groupe éliminable, et $R^2$, $R^3$ et A ont les significations indiquées ci-dessus, en présence d'une base, avec un isonitrile de formule générale

$CN-CH_2-R^1$ XX

dans laquelle $R^1$ a les significations indiquées ci-dessus, et

d) si on le désire, on convertit un composé ainsi obtenu, répondant à la formule I, en un sel formé par addition avec un acide acceptable pour l'usage pharmaceutique.

**2.** Procédé selon revendication 1, caractérisé en ce que A représente le groupe (1).

**3.** Procédé selon revendication 1 ou 2, caractérisé en ce que $R^2$ représente l'hydrogène et $R^3$ un groupe alkyle en C 1-C 7, ou bien $R^2$ et $R^3$ forment ensemble un groupe diméthylène ou triméthylène, et le composé est en configuration (S) par rapport à l'atome de carbone $\gamma$ lorsque $R^2$ et $R^3$ forment ensemble un groupe diméthylène ou triméthylène.

**4.** Procédé selon revendication 1, caractérisé en ce que l'on prépare la 7-chloro-3-(3-cyclopropyl-1,2,4-oxadiazole-5-yl)-5,6-dihydro-5-méthyl-4H-imidazo [1,5-a] [1,4[benzodiazépine-6-one.

**5.** Procédé selon revendication 1, caractérisé en ce que l'on prépare la (S)-8-chloro-1-(3-cyclopropyl-1,2,4-oxadiazole-5-yl)-12,12a-dihydro-9H,11H-azéto[2,1-c] imidazo[1,5-a] [1,4]-benzodiazépine-9-one.

**6.** Procédé selon revendication 1, caractérisé en ce que l'on prépare la (S)-8-chloro-1-(3-cyclopropyl-1,2,4-oxadiazole-5-yl)-11,12,13,13a-tétrahydro-9H-imidazo[1,5-a]pyrrolo[2,1-c] [1,4]benzodiazépine-9-one.

**7.** Procédé selon revendication 1, caractérisé en ce que l'on prépare la 3-(3-cyclopropyl-1,2,4-oxadiazole-5-yl)-8-fluoro-5,6-dihydro-5-méthyl-4H-imidazo[1,5-a] [1,4]benzodiazépine-6-one.